Europäisches Patentamt

European Patent Office

Office européen des brevets

(11) Numéro de publication: **0 356 326**
A1

# (12) DEMANDE DE BREVET EUROPEEN

(21) Numéro de dépôt: 89402307.6

(22) Date de dépôt: 18.08.89

(51) Int. Cl.5: **A 01 N 33/08**
A 61 K 9/22

(30) Priorité: 19.08.88 FR 8811041

(43) Date de publication de la demande:
28.02.90 Bulletin 90/09

(84) Etats contractants désignés:
BE CH DE ES FR IT LI NL

(71) Demandeur: COMPAGNIE FRANCAISE DE PRODUITS INDUSTRIELS
28, Boulevard Camélinat B.P. 75
F-92233 Gennevilliers Cédex (FR)

(72) Inventeur: Schapira, Joseph
32, rue Miollis
F-75015 Paris (FR)

Vincent, Jacques
21 F rue des Closeaux
F-78750 Mareil Marly (FR)

Schild, Jacques
9 Rue Léonard de Vinci
F-92230 Gennevilliers (FR)

Gamet, Jean-Claude
36 Rue Paul-Vaillant Couturier
F-18400 St Florent Sur Cher (FR)

(74) Mandataire: Koch, Gustave et al
Cabinet PLASSERAUD 84, rue d'Amsterdam
F-75009 Paris (FR)

(54) Préparation d'un désinfectant propre à prévenir la mammite subclinique chez les ruminants.

(57) Application à la préparation d'un désinfectant propre à prévenir la mammite subclinique chez les ruminants et en particulier chez la vache, d'au moins un composé de formule
R - X - CH2 - CH(OH) - CH2NH2    (I)
dans laquelle
R est un radical aliphatique en $C_5$ à $C_{18}$ à chaîne droite saturée ou insaturée,
X représente -O-, -S-, -NH- ou -CH2-,
et/ou d'au moins un de ses sels, esters et sels d'alcoyle ammonium quaternaire, étant entendu que, lorsque X est égal à -CH2-, R ne représente pas un radical en $C_{12}$.

EP 0 356 326 A1

**Description**

## PREPARATION D'UN DESINFECTANT PROPRE A PREVENIR LA MAMMITE SUBCLINIQUE CHEZ LES RUMINANTS

L'invention concerne la préparation d'un désinfectant propre à prévenir la mammite subclinique chez les ruminants et en particulier chez la vache.

La mammite subclinique est un état pathologique latent du pis qui ne se traduit par aucun signe clinique visible mais qui a pour conséquence une perte du rendement en lait ainsi qu'une détérioration de la qualité leucocytaire du lait.

Pour évaluer le degré d'infection, on peut avoir recours à des tests du genre de ceux mettant en oeuvre une réaction de coagulation pratiquement proportionnelle à la charge leucocytaire; on trouve dans le commerce, entre autres, le kit commercialisé par la Société Demanderesse sous la marque GALO-TEST® et qui permet de réaliser l'évaluation en question.

L'hygiène du pis, avant et après la traite, constitue l'un des moyens les plus sûrs pour éviter les mammites et leurs conséquences sur le lait, conséquences qui sont liées à la qualité microbienne et leucocytaire du lait et qui peuvent se traduire par des difficultés au moment de la pasteurisation et de la fermentation.

Pour assurer l'hygiène du pis immédiatement arès la traite, il a été conseillé d'utiliser des produits à base d'agents germicides appliqués par trempe ou par pulvérisation, l'agent germicide mis en oeuvre devant satisfaire simultanément à plusieurs conditions, à savoir:

- présenter un spectre germicide le plus large possible englobant aussi bien des germes dits "de réservoir" (Staphylococcus aureus, Streptococcus Agalactiae et Dysagalactiae) qui se trouvent habituellement sur le trayon, c'est-à-dire l'extrémité du pis, que les autres germes variés de l'environnement,

- présenter un pouvoir antiseptique suffisamment persis tant pour éviter les recontaminations entre les traites qui sont espacées de 8 à 12 heures,

- présenter un pouvoir irritant le moins élevé possible, et

- être sans conséquence nocive sur la santé humaine et sur la possibilité de conduire des fermentations avec le lait, dans la mesure où il est difficile d'éviter qu'on le retrouve pour le moins à l'état de traces dans le lait par la mise en contact au niveau du trayon.

Enfin, du point de vue économique, il doit être d'un prix de revient raisonnable.

Aucun des agents germicides proposés et qui comprennent:

- les dodécylbenzène sulfonates,
- les chloroisocyamurates,
- la chlorhexidine,
- les sels d'ammonium quaternaire,
- l'iode et les iodophores,
- l'hypochlorite de sodium,
- les alcoylaminoacides ou autres amphotères,

ne répond à l'ensemble des conditions énumérées ci-dessus, chacun présentant au contraire un ou plusieurs des inconvénients qu'il s'agit précisément d'éviter.

L'invention a donc pour but, surtout, de remédier à cette situation.

Et il est du mérite de la Société Demanderesse d'avoir trouvé que, pour ce faire, il convenait d'avoir recours aux composés de formule:

$$R - X - CH_2 - CH(OH) - CH_2NH_2 \quad (I)$$

dans laquelle

R est un radical aliphatique en $C_5$ à $C_{18}$ à chaîne droite saturée ou insaturée,

X représente -O-, -S-, -NH- ou -CH$_2$-,

ainsi qu'à leurs sels, esters et sels d'alcoyle ammonium quaternaire, étant entendu que, lorsque X est égal à -CH$_2$-, R ne représente pas un radical en $C_{12}$, et plus particulièrement au décyloxy-3-hydroxy-2-amino-1-propane, notamment sous la forme de son chlorhydrate, dont la formule générale est

$$C_{10}H_{21}-OCH_2-CHOH-CH_2NH_2, HCl$$

et qui se présente sous forme d'une poudre blanche, soluble dans l'eau et l'alcool éthylique.

Les composés de formule (I) et plus particulièrement le chlorhydrate de décyloxy-3-hydroxy-2-amino-1-propane ainsi que leur procédé de préparation sont décrits dans le brevet français N° 75 05647.

L'invention a donc pour objet l'application des composés de formule (I) et plus particulièrement du chlorhydrate de décyloxy-3-hydroxy-2-amino-1-propane à la préparation d'un désinfectant propre à prévenir la mammite subclinique chez les ruminants et en particulier chez la vache.

La Société Demanderesse a plus particulièrement étudié l'application du chlorhydrate de décyloxy-3-hydroxy-2-amino-1-propane.

Sa solution à 1% dans l'eau est tensio-active (27 dynes/cm environ) et présente un pH de 4 à 6 qui correspond au meilleur pH pour la peau.

Les activités bactéricide et virucide de ce produit sont décrites dans notamment la thèse de Docteur-Vétérinaire de D. LEFAY soutenue le 30 avril 1987.

Son inocuité au niveau de la peau et des muqueuses et sa très faible toxicité par voie orale ont été rapportées notamment dans la thèse référencée ci-dessus.

EP 0 356 326 A1

Plus particulièrement, l'invention a pour objet l'application des composés de formule (I) et notamment du chlorhydrate de décyloxy-3-hydroxy-2-amino-1-propane à la préparation d'un désinfectant propre à prévenir la mammite sub-clinique chez les ruminants, ladite préparation consistant à constituer une composition comportant, non seulement un composé de formule (I) et notamment le chlorhydrate de décyloxy-3-hydroxy-2-amino-1-propane, mais de plus éventuellement au moins certains des constituants suivants:

- un ou plusieurs agents désinfectants du groupe comprenant la chlorhexidine, les dérivés de biguanide et notamment le chlorhydrate de polyhexaméthylène de biguanide, les dérivés d'ammonium quaternaire, le tribromosalicylanilide, les agents désinfectants à réaction amphotère et notamment les alkylaminoacides,
- un ou plusieurs agents tensio-actifs, de préférence non ioniques, cationiques ou amphotères,
- un ou plusieurs agents émollients du type polyol et notamment le glycérol, les polyglycols, le sorbitol, la lanoline et ses dérivés,
- un ou plusieurs agents filmogènes et/ou épaississants et notamment la polyvinylpyrrolidone, les dérivés de cellulose, les polymères acryliques, les copolymères d'anhydride maléique, les gommes et les polymères extraits de produits naturels.

Le désinfectant préparé conformément à l'invention contient de 0,1 à 5% en poids et, de préférence, de 0,5 à 2%, de composé de formule (I) et notamment de chlorhydrate de décyloxy-3-hydroxy-2-amino-1-propane par rapport au poids total du désinfectant; la présence d'un ou de plusieurs des agents bactéricides indiqués plus haut permet de réduire la concentration en composé de formule (I), étant entendu toutefois que la proportion d'agent bactéricide ne devra pas être telle qu'apparaissent les inconvénients inhérents à ces produits et également rappelés plus haut.

La concentration en agent émollient est de 1% à 30%, préférentiellement de 2 à 10% en poids par rapport au poids total du désinfectant.

La présence de l'agent filmogène est souhaitable, le temps de contact et la persistance sur le pis du désinfectant étant des critères importants d'efficacité; on a avantageusement recours aux dérivés de cellulose, notamment la carboxyméthylcellulose et hydroxyéthylcellulose, à l'alcool polyvinylique, à la polyvinylpyrrolidone, aux polymères acryliques, aux copolymères d'anhydride maléique, aux gommes de xanthane, de guar, de caroube, et aux carrhagénanes.

La concentration en agent filmogène est en général de 0,1 à 6% en poids, de préférence de 0,2 à 2% par rapport au poids total du désinfectant.

Parmi les agents tensio-actifs, les non-ioniques sont préférés car ils irritent moins la peau.

Leur concentration dans le désinfectant est de 0,1 à 5% en poids et, de préférence, de 0,5 à 3% par rapport au poids total du désinfectant.

La formulation constitutive du désinfectant est complétée par de l'eau, de préférence déminéralisée, et/ou éventuellement par un ou plusieurs alcools (de façon par exemple à conférer une bonne tenue au froid), par un colorant et/ou par un parfum.

Il s'agit alors d'un désinfectant liquide.

Plus particulièrement, le désinfectant préparé conformément à l'invention comprend, pour 1 partie en poids de composé de formule (I) et plus particulièrement de chlorhydrate de décyloxy-3-hydroxy-2-amino-1-propane et, éventuellement, au moins l'un des constituants supplémentaires suivants:

- de 1 à 10 parties en poids d'agent émollient,
- de 0,1 à 10 parties en poids d'agent filmogène,
- de 0,1 à 5 parties en poids d'agent tensio-actif à activité mouillante et/ou de nettoyage et/ou antimousse,
- de 1 à 40 parties en poids d'alcool et
- de 0,1 à 5 parties de parfum et/ou de colorant,

le complément à 100 étant obtenu par de l'eau éventuellement déminéralisée.

Dans certains cas, le désinfectant se présente avantageusement sous la forme d'une pommade, d'un gel ou d'un aérosol, préparés avec les constituants habituels à ce type de produit.

La composition d'une pommade ou crème peut être avantageusement la suivante:

| | |
|---|---|
| vaseline | 16 parties en poids |
| alcool cétylique | 16 parties en poids |
| alcool laurique polyoxyéthyléné | 6 parties en poids |
| chlorhydrate de décyloxy-3-hydroxy-2-amino-1-propane | 0,2 parties en poids |
| propylèneglycol | 8 parties en poids |
| conservateur | q.s.p. |
| eau | 53,8 parties en poids |

La composition d'un gel peut être avantageusement la suivante:

| | |
|---|---|
| sorbitol | 4 parties en poids |
| Carbopol 940 | 0,3 parties en poids |
| chlorhydrate de décyloxy-3-hydroxy-2-amino-1-propane | 0,2 parties en poids |
| alcool éthylique | 23,7 parties en poids |
| eau | 71,2 parties en poids |
| alcool oléique polyoxyéthyléné | 0,3 parties en poids |
| TEA (triéthanolamine) | 0,3 parties en poids |

La composition d'un aérosol peut être avantageusement la suivante:

| | |
|---|---|
| chlorhydrate de décyloxy-3-hydroxy-2-amino-1-propane | 0,2 parties en poids |
| alcool éthylique | 79,8 parties en poids |
| gaz propulseur | 20 parties en poids |

Il peut être avantageux de commercialiser le désinfectant préparé conformément à l'invention sous la forme d'un concentré comprenant de 5 à 30% en poids de composé de formule (I), notamment de chlorhydrate de décyloxy-3-hydroxy-2-amino-1-propane. Un tel concentré, pour être applicable, est dilué avec 5 à 30 fois son poids d'eau.

La Société Demanderesse a préparé, à titre d'exemples non limitatifs, un certain nombre de désinfectants conformes à l'invention et dont la composition résulte du tableau I ci-après.

TABLEAU I

| Exemples | Constituants (proportion en % en poids) | | | | | | | | |
|---|---|---|---|---|---|---|---|---|---|
| | A | B | C | D | E | F | G | H | I |
| 1 | 92,5 | 1 | - | - | 5 | - | 1 | 0,5 | - |
| 2 | 87,22 | 0,5 | 0,28 | - | 10 | - | 1 | - | 1 |
| 3 | 87,6 | 0,5 | 0,2 | 0,2 | 10 | - | 1 | 0,5 | - |
| 4 | 86,5 | 2 | - | - | 10 | - | 1 | 0,5 | - |
| 5 | 86,5 | 2 | - | - | - | 10 | 1 | 0,5 | - |
| 6 | 86,1 | 2 | 0,2 | 0,2 | - | 10 | 1 | 0,5 | - |

A eau

B chlorhydrate de décyloxy-3-hydroxy-2-amino-1-propane

C gluconate de chlorhexidine

D chlorhydrate de polyhexaméthylène biguanide

E glycérine

F PEG 400

G nonylphénol éthoxylé

H hydroxyéthyl cellulose

I polyvinylpyrrolidone.

Les meilleurs résultats ont été enregistrés avec les désinfectants selon les exemples 1 et 3.

En effet, les compositions 1 et 3 ont été trouvées efficaces sur la souche Staphylococcus aureus selon la norme française NF T 72151 pour une dose de 0,1% de matière active, soit respectivement une dilution des compositions de 10 fois et de 5 fois.

4

**Revendications**

1. Application à la préparation d'un désinfectant propre à prévenir la mammite subclinique chez les ruminants et en particulier chez la vache, d'au moins un composé de formule

$R - X - CH_2 - CH(OH) - CH_2NH_2$ (I)

dans laquelle

R est un radical aliphatique en $C_5$ à $C_{18}$ à chaîne droite saturée ou insaturée,

X représente -O-, -S-, -NH- ou -CH$_2$-,

et/ou d'au moins un de ses sels, esters et sels d'alcoyle ammonium quaternaire, étant entendu que, lorsque X est égal à -CH$_2$-, R ne représente pas un radical en $C_{12}$.

2. Application à la préparation d'un désinfectant propre à prévenir la mammite subclinique chez les ruminants et en particulier chez la vache, du décyloxy-3-hydroxy-2-amino-1-propane, en particulier sous la forme de son chlorhydrate.

3. Application selon l'une des revendications 1 et 2, la préparation du désinfectant consistant à constituer une composition comportant, non seulement le composé de formule (I) et notamment le chlorhydrate de décyloxy-3-hydroxy-2-amino-1-propane, mais de plus éventuellement au moins certains des constituants suivants:

- un ou plusieurs agents désinfectants du groupe comprenant la chlorhexidine, les dérivés de biguanide et notamment le chlorhydrate de bromhexaméhylène, les dérivés d'ammonium quaternaire, le tribromosalicylanilide, les agents désinfectants à réaction amphotère et notamment les alkylaminoacides,

- un ou plusieurs agents tensio-actifs, de préférence non ioniques, cationiques ou amphotères,

- un ou plusieurs agents émollients du type polyol et notamment le glycérol, les polyglycols, le sorbitol, la lanoline et ses dérivés,

- un ou plusieurs agents filmogènes et/ou épaississants et notamment la polyvinylpyrrolidone, les dérivés de cellulose, les polymères acryliques, les copolymères d'anhydride maléique, les gommes et les polymères extraits de produits naturels.

4. Application selon l'une des revendications 1 à 3, le désinfectant ainsi préparé contenant de 0,1 à 5% en poids et, de préférence, de 0,5 à 2%, de composé de formule (I) et notamment de chlorhydrate de décyloxy-3-hydroxy-2-amino-1-propane par rapport au poids total du désinfectant.

5. Application selon l'une des revendications 3 et 4, le désinfectant ainsi préparé contenant de 1% à 30%, préférentiellement de 2 à 10% en poids d'agent émollient par rapport au poids total du désinfectant.

6. Application selon l'une des revendications 3 à 5, le désinfectant ainsi préparé contenant de 0,1 à 6%, de préférence de 0,2 à 2% en poids d'agent filmogène par rapport au poids total du désinfectant.

7. Application selon l'une des revendications 3 à 6, le désinfectant ainsi préparé contenant de 0,1 à 5%, de préférence de 0,5 à 3% en poids d'agent tensio-actif par rapport au poids total du désinfectant.

8. Application selon l'une des revendications 3 et 4, le désinfectant ainsi préparé contenant, pour 1 partie de composé de formule (I) et plus particulièrement de chlorhydrate de décyloxy-3-hydroxy-2-amino-1-propane, au moins l'un des constituants supplémentaires suivants:

- de 1 à 10 parties en poids d'agent émollient,

- de 0,1 à 10 parties en poids d'agent filmogène,

- de 0,1 à 5 parties en poids d'agent tensio-actif à activité mouillante et/ou de nettoyage et/ou antimousse,

- de 1 à 40 parties en poids d'alcool et

- de 0,1 à 5 parties de parfum et/ou de colorant,

le complément à 100 étant obtenu par de l'eau éventuellement déminéralisée.

# RAPPORT DE RECHERCHE EUROPEENNE

EP  89 40 2307

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | FR-A-2 301 235  (LABORATOIRES PHARMASCIENCE)<br>* Page 7, lignes 21-35; page 8, lignes 1-9; revendications 1,3-6 *<br>--- | 1-8 | A 01 N   33/08<br>A 61 K    9/22 |
| Y | EP-A-0 212 866  (MINNESOTA MINING AND MANUFACTURING CO.)<br>* Revendications 1,3-4,7,9 *<br>----- | 1-8 | |

DOMAINES TECHNIQUES RECHERCHES (Int. Cl.5)

A 01 N
A 61 K

Le présent rapport a été établi pour toutes les revendications

| Lieu de la recherche | Date d'achèvement de la recherche | Examinateur |
|---|---|---|
| LA HAYE | 05-10-1989 | RAVANEL C.M. |

EPO FORM 1503 03.82 (P0402)